# EUROPEAN PATENT SPECIFICATION

(11) **EP 2 968 072 B1**
(45) Date of publication and mention of the grant of the patent: **12.09.2018**
(21) Application number: 14716160.8
(22) Date of filing: 13.03.2014
(51) Int. Cl.: A61J 7/00, A61M 31/00

(54) **CHILD RESISTANT COVER FOR ORAL DOSAGE FORMS**
KINDERSICHERER DECKEL FÜR ORALE DARREICHUNGSFORMEN
COUVERCLE À L'ÉPREUVE DES ENFANTS POUR FORMES DOSIFIÉES ORALES

(30) Priority: 14.03.2013 US 201361781966 P
(43) Date of publication of application: 20.01.2016
(73) Proprietor: Cephalon, Inc., Frazer, PA 19355 (US)
(72) Inventor: BRADWAY, Randy J., Wyncote, Pennsylvania 19095 (US); FRANK, Gerald A., Coatesville, Pennsylvania 19320 (US)
(74) Representative: Carpmaels & Ransford LLP
(86) International application number: PCT/US2014/025483
(87) International publication number: WO 2014/159934

(56) References cited:
- US-A- 2 017 783
- US-A- 2 469 399
- US-A- 4 474 308
- US-A- 5 399 162
- US-A1- 2009 142 727
- US-B2- 7 744 558

## Description

### FIELD OF THE INVENTION

The present invention relates to a child resistant safety cover for an oral dosage form, particularly an oral dosage form that is equipped with a holder or a handle.

### BACKGROUND OF THE INVENTION

Several drug products currently on the market are provided in a form for oral administration, wherein the oral drug form is equipped and used with a holder or handle. A user of such an oral drug product could potentially remove it from its packaging and leave it unattended with the drug product exposed and accessible to another person, e.g., a child. It would be beneficial to provide a safety device for such a drug product to prevent another person, particularly a child, from inadvertently accessing the medicinal agent.

US 2,017,783 discloses a capsule administering device for holding a capsule and administering it into the throat of an animal.

US 2,469,399 discloses a disposable dispensing device for dispensing a measured amount of a liquid to a patient.

US 2009/0142727 A1 discloses a device for delivering medicinal implants into a dental pocket of a patient.

US 7,744,558 discloses a pill plunger for administering medication in pill, powder and liquid form.

US 4,474,308 discloses a tablet or pill ejector consisting of at least three detachably secured sections in the general shape of a pen. The front end of the device is provided with a hole communicating with axially aligned bores formed longitudinally in the sections and through which a pill may be ejected quickly. An ejector rod is provided in the bores adapted to move from a cocked position to a released position wherein it passes instantly through a pill retaining member located in the front section of the device. Cocking and trigger mechanisms are provided. In an alternate embodiment the front section is provided with a revolvable barrel adapted to contain a plurality of pills for ejection. The rear end of the device may be provided with a storage compartment and cap.

US 5,399,162 discloses an automatic balling gun which includes an elongated member having a tubular profile which is easily operated and manipulated by the single hand of the user. A release assembly is mounted along the length of the tubular member to dispense medicine orally to an animal by merely depressing a plunger. A knob is provided on a proximal end of the automatic balling gun which facilitates cocking the gun and loading a dispensing chamber thereof with a capsule or pill to be dispensed. The automatic balling gun can then be inserted into the animal to be treated followed by depressing the plunger on the release assembly to dispense the medication from the gun.

### SUMMARY OF THE INVENTION

The present invention provides a child resistant safety device for an oral dosage form as recited in the claims.

According to some embodiments of these aspects of the invention, the position (or configuration) of the device or retractable oral dosage form, which is referred to herein as a "retracted position" (or "retracted configuration") corresponds to a "retracted/locked position," (or "retracted/locked configuration"), wherein a releasable locking mechanism prevents movement to the deployed position until and unless the locking mechanism is released.

### BRIEF DESCRIPTION OF THE FIGURES

The invention is best understood from the following detailed description when read in connection with the accompanying drawing. It is emphasized that, according to common practice, the various features of the drawing are not to scale. Included in the drawing are the following figures:
FIG. 1A depicts a perspective view of a safety device for an oral dosage form according to a first exemplary embodiment of the invention, wherein the safety device is shown in a retracted configuration whereby the oral dosage form is shielded by a child resistant cover.
FIG. 1B depicts a perspective view of the safety device of FIG. 1A shown in a deployed configuration whereby the oral dosage form is exposed.
FIG. 2 depicts an exploded view of the safety device of FIGs. 1A and 1B.
FIGs. 3 and 4 depict right side elevation and front elevation views, respectively, of the safety device of FIGs. 1A and 1B shown in a retracted configuration.
FIG. 5 depicts a cross-sectional side elevation view of the retracted safety device of FIG. 3 taken along the lines 5-5.
FIG. 6 depicts a cross-sectional side elevation view of the retracted safety device of FIG. 4 taken along the lines 6-6.
FIGs. 7A and 7B depict front elevation and cross-sectional views, respectively, of the safety device of FIG. 6 with the tabs of the child resistant cover shown in a depressed position.
FIGs. 8A and 8B depict elevation and cross-sectional views, respectively, of the safety device of FIGs. 7A and 7B with the tabs of the child resistant cover shown depressed and the child resistant cover slid in a proximal direction.
FIGs. 9A and 9B depict elevation and cross-sectional views, respectively, of the safety device of FIGs. 8A and 8B with the tabs of the child resistant cover depressed and the child resistant cover slid further in the proximal direction such that the oral dosage form is exposed.
FIGs. 10 and 11 depict perspective and side elevation views, respectively, of the child resistant cover of the safety device of FIGs. 1-9.
FIG. 12 depicts a cross-sectional side elevation view of the child resistant cover of FIG. 11 taken along the lines 12-12.
FIG. 13 depicts an enlarged cross-sectional plan view of the child resistant cover of FIG. 11 taken along the lines 13-13.
FIGs. 14-16 depict perspective, right side and front elevation views, respectively, of the distal shaft portion of the safety device of FIGs. 1-9.
FIG. 17 depicts a cross-sectional side elevation view of the distal shaft portion of FIG. 15 taken along the lines 17-17.
FIG. 18A depicts a front perspective view of a safety device for an oral dosage form according to a second exemplary embodiment of the invention, wherein the safety device is shown in a retracted configuration whereby the oral dosage form is shielded by a child resistant cover of the safety device.
FIG. 18B depicts a right side perspective view of the safety device of FIG. 18A, wherein the internal mechanisms of the safety device are shown in phantom lines.
FIG. 18C depicts a front perspective view of the safety device of FIG. 18A,
wherein the safety device is shown in a deployed configuration whereby the oral dosage form is exposed.

### DETAILED DESCRIPTION OF THE INVENTION

This invention will now be described with reference to several embodiments selected for illustration in the drawings. It will be appreciated that the scope of the invention is are not limited to the illustrated embodiments.

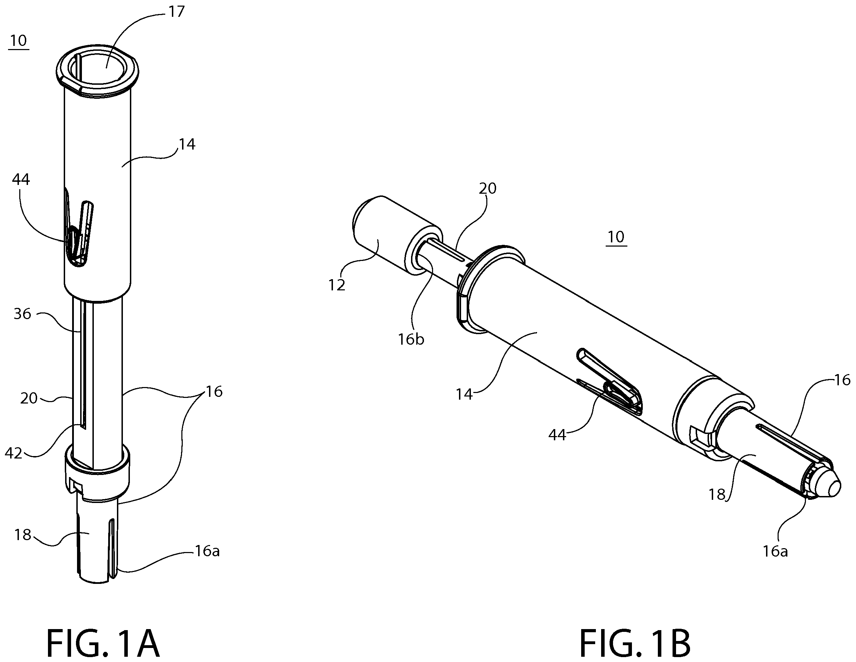

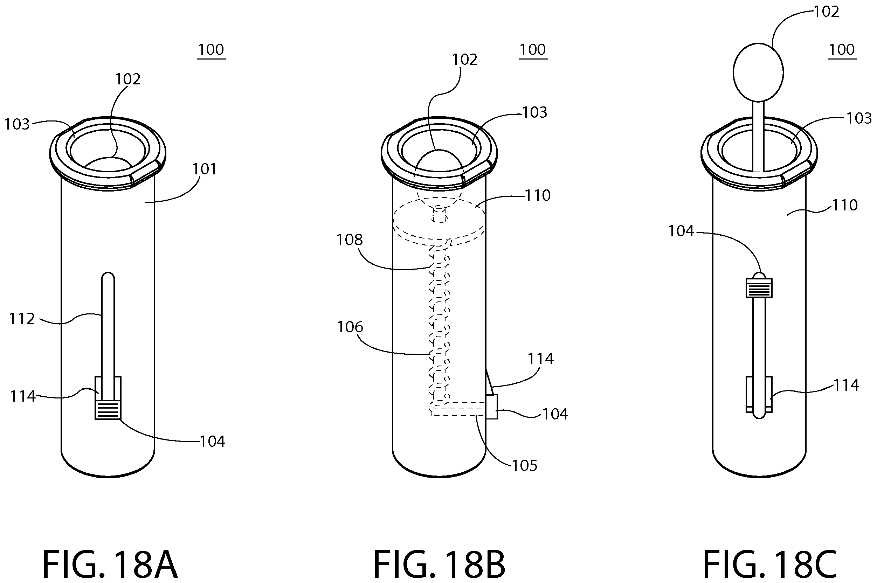

A first exemplary embodiment of a child resistant safety device for an oral dosage form comprising a medicinal agent is depicted in FIGs. 1A-17, and a second exemplary embodiment of a child resistant safety device for an oral dosage form comprising a medicinal agent is depicted in FIGs. 18A through 18C.

The expression "child resistant," as used herein, relates to special packaging that is used to reduce the risk of children ingesting dangerous items or substances. Packaging designated as "child resistant" is typically subject to the rigorous testing set out in the regulatory scheme of 16 C.F.R. § 1700, which provides specific protocols for performance testing with actual children, to determine whether certain packaging can be opened. The oral dosage form described herein may comprise, for example, a solid, or a permeable or semi-permeable matrix capable of drug elution, form. The oral dosage form may be, for example, a lozenge, a pill, a tablet, or other oral dosage form capable of drug delivery via the buccal or sublingual route (transmucosal), in particular, in a form that can be equipped and used with a holders or handle.

The oral dosage form comprises at least one medicinal agent. While at least one medicinal agent is required, it is contemplated that multiple medicinal agents may also be used. The term "medicinal agent" refers generally to drug products. Such medicinal agents may include pharmaceutical ingredients, vitamins, minerals, and dietary supplements and combinations thereof. Pharmaceutical ingredients may include, for example, antacids, analgesics, stimulants, sleep aids, hypnotics, antipyretics, antimicrobials, anxiolytics, laxatives, antidepressants, antidiuretics, antiflatuents, antispasmodics, anti-inflammatory, antibiotics, diuretics, anorexics, antihistamines, antiasthmatics, antidiuretics, antiflatuents, antimigraine agents, antispasmodics, sedatives, antihyperactives, antihypertensives, tranquilizers, decongestants, immunosuppressants, anticancers, antivirals, antiparasitics, antifungals, antiemetics, antidepressants, antiepileptics, local anesthetics, vasoactive agents, antiasthmatics, skeletal muscle relaxants, drugs for parkinsonism, antipsychotics, hematopoietic growth factors, antihyperlipidemics, anticoagulants, fibrinolytics, antithrombotics, hormones, therapeutic proteins and peptides, antiarrhythmia, antiangina, beta blockers and combinations thereof.

Also included as medicinal agents that may be administered using the present invention are the drugs and pharmaceutically active ingredients described in Mantelle, U.S. Pat. No. 5,234,957, in columns 18 through 21.

According to some embodiments of the invention, the medicinal agent is a pharmaceutical agent having a high likelihood of abuse by people. In some embodiments of the invention, the medicinal agent is a pain medication such as a narcotic or non-narcotic analgesic, for example, as listed on pages THER-2 and THER-3 of The Merck Index, 13th Ed., Published by Merck & Co., Inc., of Whitehouse Station, N.J., copyright 2001.

Suitable narcotic analgesics include, for example, analgesics, pain relievers, opioids, such as, for example, oxycodone, codeine, hydrocodone, morphine, hydromorphone, oxymorphone, methadone, propoxyphene, meperidine, fentanyl, buprenorphine, butorphanol, dezocine, levomethadyl acetate, levorphanol, nalbuphine, pentazocine, remifentanil, sufentanil, tramadol; stimulants, such as, for example, amphetamine, methamphetamine, dexamphetamine, methylphenidate, dexmethylphenidate, and pemoline; sedative / hypnotics, such as, for example, barbiturates, such as, for example, amobarbital, aprobarbital, butabarbital, mephobarbital, phenobarbital, secobarbital; benzodiazepines, such as, for example, alprazolam, clonazepam, diazepam, estazolam, flurazepam, halazepam, lorazepam, midazolam, quazepam, temazepam, triazolam, prazepam, and oxazepam; and eugeroics, such as, for example, modafinil and armodafinil. Particularly suitable medicinal agents include oxycodone, fentanyl and hydromorphone. Salts of all of the medicinal agents listed above are also contemplated, as are their stereogenic isomers, polymorphs and solvates.

The medicinal agent may be administered via transmucosal or sublingual routes.

Referring now to the first exemplary embodiment of the child resistant safety device 10, FIGs. 1A and 1B each depicts a perspective view of a child resistant safety device 10 for an oral dosage form 12. In FIGs. 1A and 3-6, the child resistant safety device 10 is shown in a retracted configuration wherein the oral dosage form 12 is shielded by a child resistant cover 14. In FIG. 1B, the child resistant safety device 10 is shown in a deployed configuration wherein the oral dosage form 12 is exposed through an opening 17 in the cover 14 for the purpose of consumption. As will be described hereinafter, the child resistant safety device 10 is capable of moving between the retracted configuration of FIG. 1A and the deployed configuration of FIG. 1B by a user of the device 10.

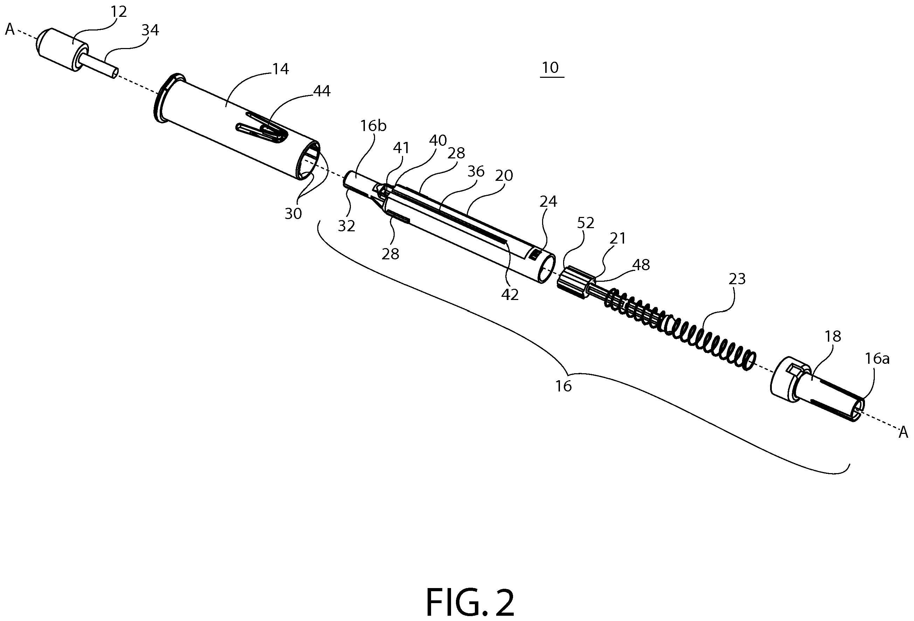

FIG. 2 depicts an exploded view of the safety device 10 of FIGs. 1A and 1B. As best shown in FIG. 2, the safety device 10 generally comprises a shaft assembly 16 and a safety cover 14 that is retractably mounted to the shaft assembly 16. The proximal end 16a of the shaft assembly 16 is configured to be grasped by a user, and the distal end 16b of the shaft assembly 16 is configured to fixedly receive the oral dosage form 12. It should be understood that the oral dosage form 12 is not necessarily a component of the safety device 10. The components of the safety device 10 may be formed from any materials known to those of ordinary skill in the art, such as plastic or metal, for example. The spring 23 of the safety device 10 may be formed from any resilient material, such as spring steel, for example.

Referring now to FIGs. 1A-6, the shaft assembly 16 includes a two-piece shaft, having a proximal shaft portion 18 that is fixed to a distal shaft portion 20, and a captivated plunger 21 that is loaded by a spring 23 and is capable of translation within an opening formed through the two-piece shaft.

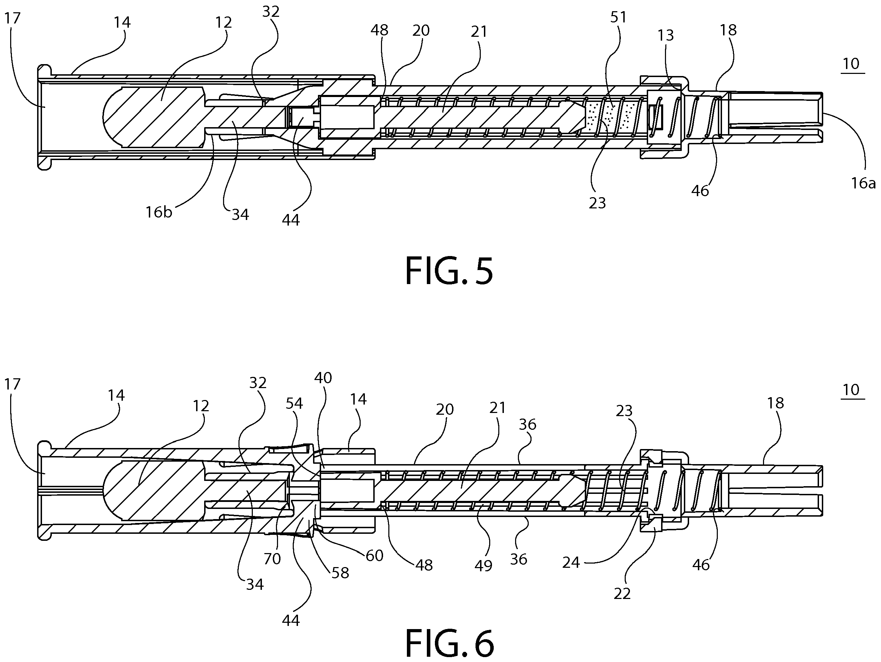

Referring now to the individual components of the shaft assembly 16, the proximal shaft portion 18 includes a substantially tubular body having an opening extending along its entire length such that both ends of the tubular body are open. As shown in FIG. 5, a circumferential shoulder 13 is formed on the distal end of the proximal shaft portion 18. In an assembled configuration of the shaft assembly 16, the proximal end of the distal shaft portion 20 is configured to be seated on the shoulder of the proximal shaft portion 18, as best shown in FIGs. 5 and 6. According to this exemplary embodiment, the shaft portions 18 and 20 of the shaft assembly 16 are separate components that are fixed to each other, however, the shaft portions 18 and 20 may be combined together into a single unitary component.

As shown in FIG. 6, a circumferential shoulder 46 is formed on the interior surface of the proximal shaft portion 18. In an assembled configuration of the shaft assembly 16, the proximal end of the spring 23 is configured to be seated on the shoulder 46 of the proximal shaft portion 18.

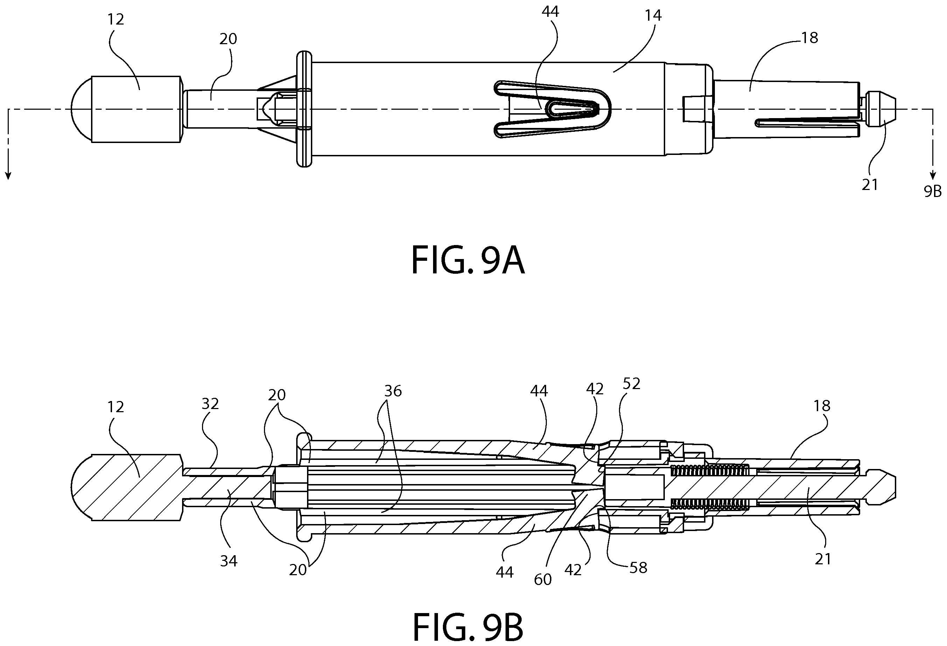

A radially-inwardly extending protrusion 22 is defined on the distal end of the proximal shaft portion 18 (see FIG. 6). In an assembled form of the shaft assembly 16, the radially-inwardly extending protrusion 22 is press-fit into a rectangular slot 24 that is defined on the exterior surface of the proximal end of the distal shaft portion 20. The shaft portions 18 and 20 are not readily releasable from each other. The opening at the proximal end 16a of the proximal shaft portion 18 is sized to accommodate the proximal end of the plunger 21 when the plunger 21 is in its fully-deployed position, as shown in FIG. 9B.

As best shown in FIGs. 2 and 14-17, the distal shaft portion 20 of the shaft assembly 16 includes a substantially tubular body having an opening extending along its entire length such that both ends of the tubular body are open. At least one guiding rib 28 extends from the outer surface of the distal shaft portion 20 in an axial direction along the longitudinal axis 'A.' The guiding ribs 28 are located toward the distal end of the distal shaft portion 20. The guiding ribs 28 are positioned on opposite sides of the distal shaft portion 20.

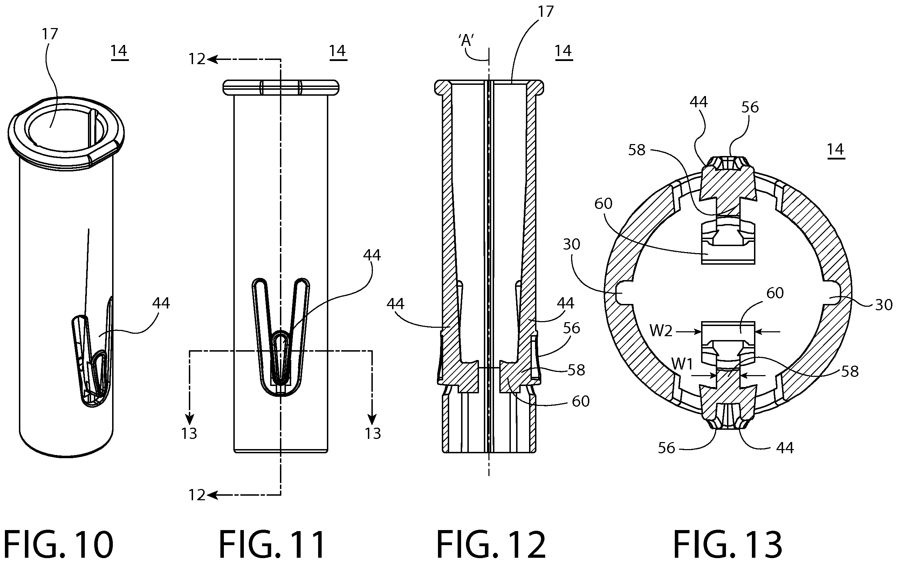

In an assembled form of the safety device 10, the guiding ribs 28 are slidingly positioned within corresponding slots 30 (see FIGs. 2 and 13) that are defined on the interior surface of the safety cover 14. The guiding ribs 28 and the slots 30 help guide the shaft assembly 16 as it translates with respect to the safety cover 14, or vice versa. The guiding ribs 28 and the slots 30 also prevent rotation of the shaft assembly 16 with respect to the safety cover 14, or vice versa, about the longitudinal axis 'A.'

The distal end 16b of the distal shaft portion 20 includes a necked-down portion 32. The post 34 of the oral dosage form 12 is fixedly positioned in the opening that passes through the necked-down portion 32. The post 34 of the oral dosage form 12 and the necked-down portion 32 may be fixed together by a sonic weld, an interference fit or an adhesive, for example, to prevent inadvertent detachment of the oral dosage form 12 from the safety device 10.

At least one slot 36 is defined along the central portion of the distal shaft portion 20. According to this exemplary embodiment, as best shown in FIG. 6, two slots 36 are positioned opposite one another on the distal shaft portion 20. Although only one slot 36 will be described hereinafter, it should be understood that the other slot 36 is both structurally and functionally equivalent. The slot 36 is defined through the entire thickness of the side wall of the distal shaft portion 20. The width 'W' of the slot 36 is constant along its length.

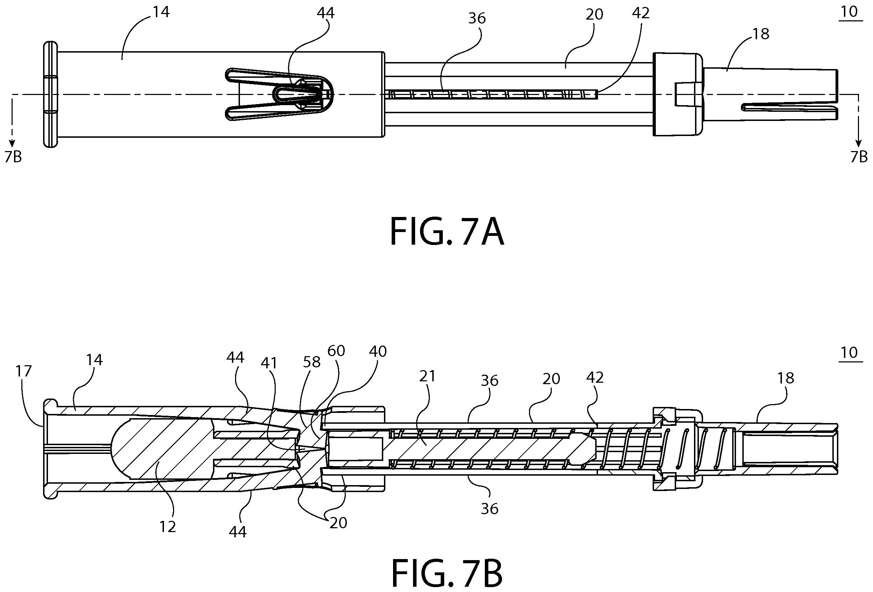

As best shown in FIGs. 2, 8B and 16, an opening 40 is provided at the distal end of the slot 36, and a wall 42 is provided at the proximal end of the slot 36. Thus, the slot 36 extends between the opening 40 and the wall 42. As will be described in greater detail with respect to FIGs. 6 and 7B, depressible and resilient tabs 44 on the cover 14 are both removably and slidably positioned with respective slots 36 on the distal shaft portion 20.

A hole 41 is defined on the outer surface of the distal shaft portion 20 at the entrance of each slot 36, i.e., at a location that is directly distal of the opening 40 of each slot 36. As will be described in greater detail with respect to FIGs. 6 and 7B, the hole 41 accommodates translation of a respective tab 44 of the cover 14 in a radial direction toward the longitudinal axis 'A' of the safety device 10. According to this exemplary embodiment, the hole 41 passes through the entirety of the distal shaft portion 20.

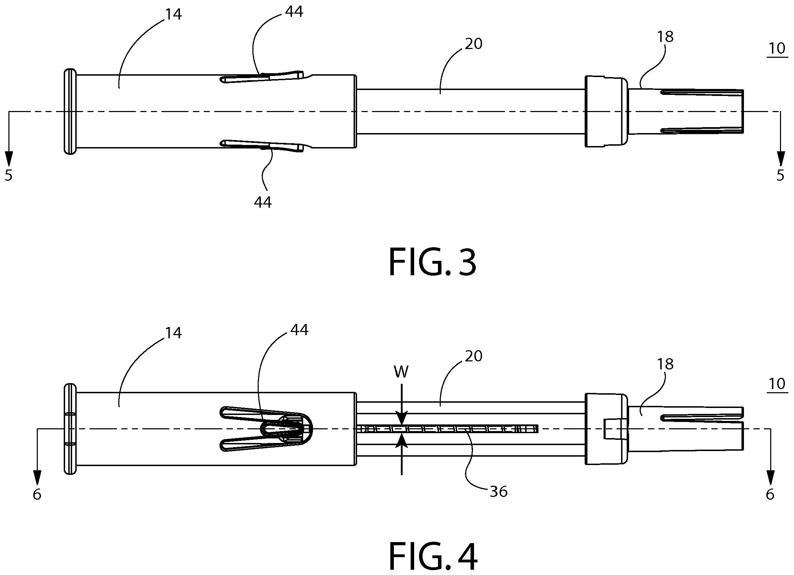

Referring now to FIGs. 2, 5 and 6, a plunger 21 of the shaft assembly 16 is slideably positioned within an interior chamber 49 of the shaft assembly 16. A resilient compression spring 23 bears on the plunger 21 and the proximal shaft portion 18. More particularly, the spring 23 is mounted between a shoulder 46 (see FIG. 6) that is formed on the interior surface of the proximal shaft portion 18 and another shoulder 48 that is formed on the exterior surface of the distal end of the plunger 21. The spring 23 biases the plunger 21 in the distal direction against the cover 14, thereby biasing the cover 14 toward its retracted position that is shown in FIGs. 3-6. The spring rate of the spring 23 is selected such that it is low enough to enable easy translation of the plunger 21 by the user, yet high enough to return the plunger 21 to its retracted position shown in FIGs. 5 and 6.

The device 10 may incorporate a damping means to prevent the device 10 from moving between the deployed and retracted positions too rapidly. The damping means may be a fluid 51 (shown schematically in FIG. 5), such as a food-grade grease, that is provided in the interior chamber 49 of the shaft assembly 16. To prevent the escapement of the fluid 51 from the interior chamber 49, the device 10 may be modified by (i) mounting an O-ring (not shown) at the proximal end of the plunger 21 to contact the revolved interior surface of the shaft assembly 16, and (ii) closing at least the proximal end 16a of the shaft assembly 16.

The damping means may also be an O-ring (not shown) that is mounted at the proximal end of the plunger 21 to contact the revolved interior surface of the shaft assembly 16. The O-ring would provide a friction force that opposes the force of the spring 23 as the spring 23 automatically translates the device 10 from a deployed position to a retracted position once the device 10 is released by the user.

The damping means may also be a second spring that is mounted to the plunger 21 to bias the plunger 21 in a proximal direction. One of ordinary skill in the art will recognize that the spring rate of the second spring would be lower than that of the spring 23 so that the device 10 can automatically return to its retracted position once the device 10 is released by the user. The damping means is an optional feature of the invention and it may be omitted entirely.

The plunger 21 is sandwiched between the spring 23 and the cover 14. The distal end 52 (see FIG. 2) of the plunger 21 bears on the proximal end of the cover 14. When a user translates the cover 14 in a proximal direction (thereby moving the device 10 from the retracted position to the deployed position), the proximal end of the cover 14 pushes the plunger 21 in the proximal direction against the force of the spring 23. When a user releases the device 10 (causing the device 10 to move from the deployed position to the retracted position), the spring 23 urges the plunger 21 in a distal direction against the cover 14, thereby causing the cover 14 to also move in the distal direction.

The child resistant cover 14 of the safety device 10 is best shown in FIGs. 1A, 2 and 10-13. Detailed views of the cover 14 are shown in FIGs. 10-13. Referring now to those figures, the cover 14 includes a tubular body defining an opening 17 at its distal end through which the medicinal agent 12 can be exposed, and at least one resiliently moveable tab 44 that is formed on the body. The cover 14 is a unitary component, according to this embodiment.

According to this exemplary embodiment, the cover 14 includes two tabs 44 that are defined on opposing sides of the body. The structure and function of only one of the tabs 44 will be described hereinafter, however, it should be understood that the structure and function of both tabs 44 are the same.

The tab 44 of the safety cover 14 interacts with the slot 36 that is formed on the shaft assembly 16 to releasably lock the safety device 10 in the retracted position. The tab 44 may also be referred to herein as a releasable locking mechanism. The tab 44 is configured to "child resistant" the device 10 because the tab 44 must be depressed inwardly toward the longitudinal axis 'A' (see FIG. 12) before the cover 14 can be translated with respect to the shaft assembly 16 (or vice versa) to expose the medicinal agent 12.

Referring now to FIGs. 12 and 13, the tab 44 includes an outer surface 56 that is capable of being contacted by the finger of a user, a relatively narrow rib 58 depending from the outer surface 56 that extends in a radial direction toward the longitudinal axis 'A,' and a wide stop 60 that is defined on the end of the narrow rib 58. The width 'W1' of the rib 58 is less than the width 'W' (see FIG. 4) of the slot 36, whereas the width 'W2' of the stop 60 is greater than the width 'W' of the slot 36. Accordingly, in the assembled form of the device 10, the rib is 58 capable of translating in the slot 36, whereas the stop 60 is not able to be positioned within the slot 36.

In the retracted position of the device 10, the stop 60 is registered with the opening 40 of the slot 36. This is referred to herein as the locked configuration of the tab 44. The stop 60 abuts against a bearing surface 54 at the distal end 16b of the shaft assembly 16 because the width 'W2' of the stop 60 is greater than the width 'W' of the opening 40 of the slot 36. Thus, the stop 60 can not pass through the opening 40. The abutment between the stop 60 and the opening 40 prevents the cover 14 from translating in a proximal direction with respect to the shaft assembly 16, and prevents the shaft assembly 16 from translating in a proximal direction with respect to the cover 14. Thus, in a retracted position of the device 10, the cover 14 is incapable of translating with respect to the shaft assembly 16 (and vice versa), thereby preventing access to the medicinal agent 12 that is contained within the interior of the cover 14. In the retracted position of the device, the narrow rib 58 does not register with the slot 36, as shown in FIG. 6.

Depressing the tab 44 towards the longitudinal axis 'A' registers the rib 58 with the opening 40 of the slot 36, and moves the stop 60 into the hole 41 that is formed on the distal shaft portion 20. Thereafter, the rib 58 can translate along the length of the slot 36 upon translating the cover 14 with respect to the shaft assembly (and vice versa), while the stop 60 travels along the interior surface of the distal shaft portion 20. This is referred to herein as the unlocked configuration of the tab 44.

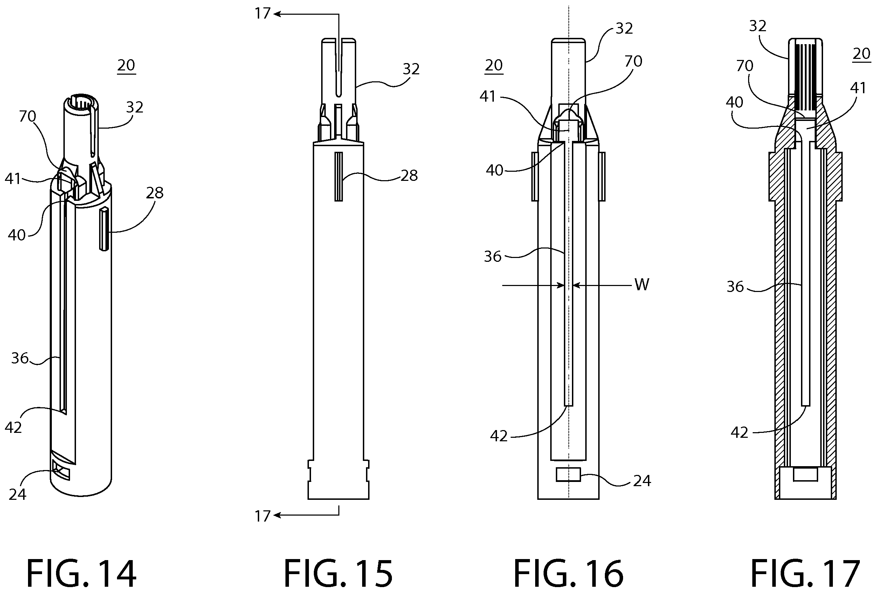

Referring now to the operation of the safety device 10, FIGS. 6, 7B, 8B and 9B depict successive movement of the safety device from a retracted position to the deployed position. Beginning at FIG. 6, the safety device 10 is ordinarily stored in the retracted position that is shown in FIG. 6. In the retracted position in FIG. 6, the cover 14 can not be translated with respect to the shaft assembly 16 (and vice versa) because the stop 60 of the tab 44 is lodged in the hole 41 of the distal shaft portion 20 and is sandwiched between the opening 40 of the slot 36 and the surface 70 (see FIG. 6 and 17). As described previously, the stop 60 is wider than the opening 40, and thus can not enter the opening 40.

To move the safety device 10 from the retracted position shown in FIG. 6 to the deployed position shown in FIG. 9B, the tab 44 must first be depressed by the user toward the longitudinal axis A. Although the operation of only one tab 44 is described in this section, it should be understood that the other tab 44 operates in the same fashion. This step of the process represents the "child resistant" aspect of the device 10. FIG. 7B depicts the depressed position of the tab 44. Depressing the tab 44 towards the longitudinal axis 'A' registers the rib 58 of the tab 44 with the opening 40 of the slot 36, and moves the stop 60 into the hole 41 that is formed on the distal shaft portion 20. As noted previously, the width of the rib 58 is less than the width of the slot 36, thus, the rib 58 can translate along the slot 36. Once the tab 44 is depressed, as shown in FIG. 7B, the cover 14 is capable of translating along the longitudinal axis 'A' with respect to the shaft assembly 16 (and vice versa).

FIGs. 7B, 8B and 9B depict the sequential movement of the safety device 10 from a retracted position to the deployed position. After the tab 44 is depressed, there are three different ways to translate the safety device 10 to the deployed position. First, the cover 14 can be translated in the proximal direction while the shaft assembly 16 remains stationary. Second, the shaft assembly 16 can be translated in a distal direction while the cover 14 remains stationary. Third, the cover 14 can be translated in the proximal direction while the shaft assembly 16 is translated in a distal direction. Any of the aforementioned translational movements will produce the same result, i.e., the safety device 10 will be moved to the deployed position of FIG. 9B. The description that follows assumes that the cover 14 is translated in the proximal direction while the shaft assembly 16 remains stationary.

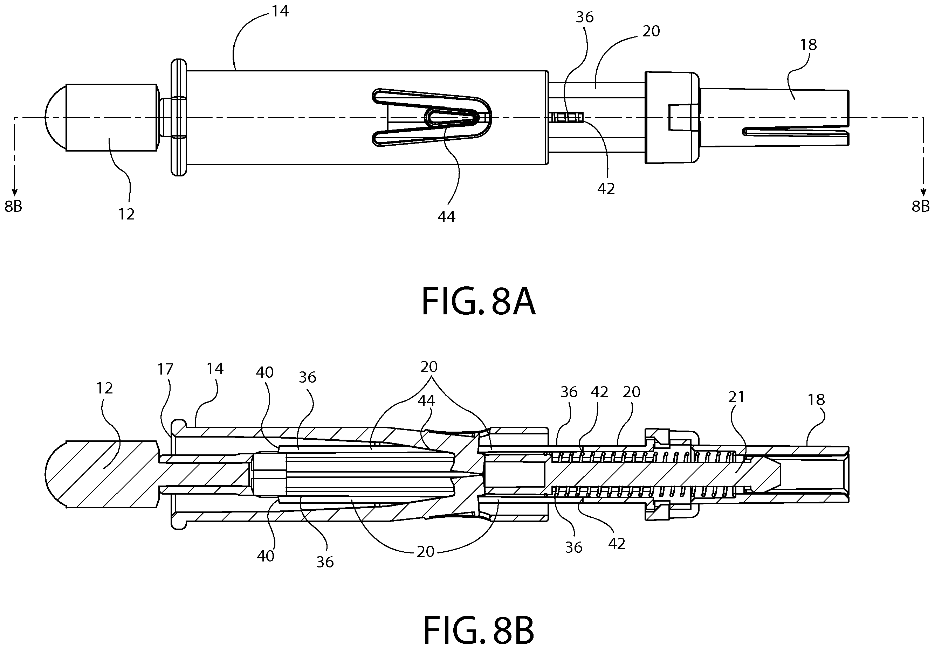

Once the tab 44 is depressed, as shown in FIG. 7B, the rib 58 of the tab 44 is registered with the opening 40 of the slot 36. Thereafter, the cover 14 is translated in a proximal direction along the longitudinal axis 'A' against the force of the spring 23 while the shaft assembly 16 remains stationary. As the cover 14 is translated in the proximal direction toward the position shown in FIG. 8B, the rib 58 slides in the slot 36 while the stop 60 of the tab 44 rides along the revolved interior surface of the distal shaft portion 20. At this point, the tab 44 can be released by the user. Upon releasing the tab 44, the rib 58 remains in the slot 36 while the stop 60 continues to bear on the revolved interior surface of the distal shaft portion 20. The stop 60 does not prevent translation of the cover 14 once the stop 60 is positioned against the revolved interior surface of the distal shaft portion 20.

As the stop 60 is translated along the interior surface of the distal shaft portion 20 toward the position shown in FIG. 8B, the proximal end of the stop 60 bears on the distal end surface 52 of the plunger 21 thereby urging the plunger 21 in the proximal direction against the force of the spring 23. Thus, the cover 14 is translated against the force of the spring 23. The spring 23 is compressed between the plunger 21 and the proximal shaft portion 18 as the user translates the cover 14 in the proximal direction.

The user translates the cover 14 in a proximal direction along the longitudinal axis 'A' until the rib 58 contacts the end wall 42 of the slot 36 at which point the cover 14 is prevented from moving further in the proximal direction. At this point, the device 10 is in the deployed position that is shown in FIG. 9B. In the deployed position of the device 10, the cover 14 is spaced from the medicinal agent 12 and the medicinal agent 12 is exposed and available to the user.

After the medicinal agent 12 is consumed (or partially consumed) by the user, the user releases the cover 14. The spring 23 expands and causes the cover 14 to automatically return to its retracted position shown in FIG. 6. As previously described, if the device 10 includes a damping agent 51 (see FIG. 5), the damping agent 51 would slow the return of the cover 14 to the retracted position. Once the cover 14 returns to its retracted position, the resilient tab 44 moves outward and away from the longitudinal axis 'A' and returns to its position shown in FIG. 6. Thereafter, the device 10 may be moved back to the deployed position, as previously described, if so desired.

FIGs. 18A-18C depict another safety device 100 for a medicinal agent, according to a second exemplary embodiment of the invention. The safety device 100 generally includes a hollow cylindrical tube 101 defining an opening 103, a spring-loaded shaft 108 that is moveably positioned within the interior of the tube 101, a medicinal agent 102 that is fixedly attached to a distal end of the shaft 108, and a child resistant lever 104 that is fixedly attached to a proximal end of the spring-loaded shaft 108.

The child resistant lever 104 is provided for moving the shaft 108 and the medicinal agent 102 between the retracted position of FIGs. 18A and 18B and the deployed position of FIG. 18C. In a retracted position of the device 100, the user is unable to consume the medicinal agent 102, whereas, in the deployed position of the device 100, the medicinal agent 102 is available to the user for consumption.

A spring 106 is positioned around the shaft 108 between an interior shoulder 110 of the tube 101 and a leg 105 extending from the lever 104. The leg 105 is fixedly connected between the lever 104 and the proximal end of the shaft 108. The spring 106 is positioned to bias the lever 104, the shaft 108 and the medicinal agent 102 in a downward direction (as viewed in FIGs. 18A-18C) toward the retracted position shown in FIG. 18B.

A ramp 114 is formed on the outer surface of the tube 101. In operation, a user must pull the lever 104 away from the tube 101 to clear the ramp 114 before advancing the lever 104 (along with the medicinal agent 102 that is indirectly attached to the lever 104) in an upward direction toward the deployed position shown in FIG. 18C. Upon releasing the lever 104, the spring 106 automatically urges the lever 104, the shaft 108 and the medicinal agent 102 in a downward direction to the retracted position shown in FIG. 14B. The force of the spring 106 is sufficient to move the lever 104 in the downward direction along the angled surface of the ramp 114. The lever 104 ultimately comes to rest in the position shown in FIG. 18B.

The ramp 114 serves as the child resistant feature of the device 100. Those of ordinary skill in the art will recognize that other ways exist to child resistant the device 100.

Although the invention is illustrated and described herein with reference to specific embodiments, the invention is not intended to be limited to the details shown. Rather, various modifications may be made in the details within the scope of the invention. For example, there are a number of alternatives for incorporating the required child resistant feature, e.g., push-and-turn, opposing digit unlocking, etc. It will further be appreciated that the drawings are not rendered to any particular proportion or scale. The invention is not limited to any particular dimensions, materials, or other details of the illustrated embodiments.

## Claims

1. A child resistant safety device (10; 100) for an oral dosage form (12; 102) comprising:
a handle comprising a shaft assembly (16; 108) having a proximal end (16a; 104) and a distal end (16b) to which an oral dosage form (12; 102) is fixedly mounted either directly or indirectly;
a cover (14; 101) that moves with respect to the handle, or vice versa, for selectively shielding the oral dosage form (12; 102), wherein, the cover (14; 101) or the handle is biased toward a retracted position in which the cover (14; 101) conceals the oral dosage form (12; 102) to limit access to the oral dosage form (12; 102), and, the cover (14; 101) or the handle is moveable to a deployed position in which the oral dosage form (12; 102) is accessible to the user; and
a releasable locking mechanism (44; 104) that is moveable between a locked configuration and an unlocked configuration, wherein, in the locked configuration, the releasable locking mechanism (44; 104) is configured to prevent movement of the cover (14; 101) or the handle to the deployed configuration, and, in the unlocked configuration, the releasable locking mechanism (44; 104) is configured to permit movement of the cover (14; 101) or the handle to the deployed configuration.

2. The child resistant safety device (10; 100) of claim 1 further comprising a spring (23; 106) that is configured to bias the child resistant safety device (10; 100) toward the retracted position.

3. The child resistant safety device (10; 100) of claim 2, wherein the spring (23; 106) is positioned to bias the cover (14; 101) and the handle in opposite directions.

4. The child resistant safety device (10; 100) of claim 2 further comprising a moveable plunger (21) positioned within the shaft assembly (16), wherein the plunger (21) is biased against the cover (14) by the spring (23).

5. The child resistant safety device (10; 100) of claim 1 further comprising a damping means (51) for damping motion of the cover (14; 101) or the handle between the retracted and deployed positions.

6. The child resistant safety device (10;100) of claim 4, wherein at least a portion of the handle is positioned within the cover (14) in both the deployed and retracted positions.

7. The child resistant safety device (10; 100) of claim 1 wherein the proximal end (16a; 104) of the handle is for grasping by a user.

8. The child resistant safety device (10; 100) of claim 7, wherein the releasable locking mechanism (44) comprises a moveable tab on the cover (14) that is configured to be engaged with the handle.

9. The child resistant safety device (10; 100) of claim 8, wherein the tab (44) includes a rib portion (58) and a stop portion (60), wherein a width of the stop portion (60) is greater than a width of the rib portion (58).

10. The child resistant safety device (10; 100) of claim 9, wherein the tab (44) is biased toward the locked configuration in which the stop portion (60) of the tab (44) abuts against a bearing surface (54) of the handle to prevent movement of the cover (14; 101) or the handle to the deployed configuration.

11. The child resistant safety device (10; 100) of claim 8, wherein the tab (44) includes a rib portion (58) and a stop portion (60), and the tab (44) is biased toward the locked configuration in which the stop portion (60) of the tab (44) abuts against a bearing surface (54) of the handle to prevent movement of the cover (14; 101) or the handle to the deployed configuration.

12. The child resistant safety device of claim 10, wherein the tab (44) is moveable to the unlocked configuration in which the stop portion (60) of the tab (44) is separated from the bearing surface (54) of the handle, and the rib portion (58) of the tab (44) is aligned with a slot (36) that is formed on the handle.

13. The child resistant safety device of claim 12, wherein the rib portion (58) is configured to move in the slot (36) of the handle to permit movement of the cover (14; 101) or the handle to the deployed configuration.

14. The child resistant safety device of claim 12, wherein a width of the slot (36) is less than the width of the stop portion (60) and greater than the width of the rib portion (58).

## Patentansprüche

1. Kindersichere Sicherheitsvorrichtung (10, 100) für eine orale Darreichungsform (12; 102), umfassend:
einen Griff, umfassend eine Schaftanordnung (16; 108) mit einem proximalen Ende (16a; 104) und einem distalen Ende (16b), womit eine orale Darreichungsform (12; 102) entweder direkt oder indirekt fest montiert ist;
einen Deckel (14; 101), der sich in Bezug auf den Griff bewegt, oder umgekehrt, zum selektiven Abschirmen der oralen Darreichungsform (12; 102), wobei der Deckel (14; 101) oder der Griff zu einer zurückgezogenen Position vorgespannt ist, in welcher der Deckel (14; 101) die orale Darreichungsform (12; 102) verdeckt, um einen Zugang zu der oralen Darreichungsform (12; 102) einzuschränken, und der Deckel (14; 101) oder der Griff zu einer ausgefahrenen Position, in der die orale Darreichungsform (12; 102) für den Benutzer zugänglich ist, bewegt werden kann; und
einen lösbaren Verriegelungsmechanismus (44; 104), der zwischen einer verriegelten Auslegung und einer unverriegelten Auslegung bewegt werden kann, wobei, in der verriegelten Auslegung, der lösbare Verriegelungsmechanismus (44; 104) ausgelegt ist, eine Bewegung des Deckels (14; 101) oder des Griffs in die ausgefahrene Auslegung zu verhindern, und, in der unverriegelten Auslegung, der lösbare Verriegelungsmechanismus (44; 104) ausgelegt ist, eine Bewegung des Deckels (14; 101) oder des Griffs in die ausgefahrene Auslegung zu gestatten.

2. Kindersichere Sicherheitsvorrichtung (10; 100) nach Anspruch 1, ferner umfassend eine Feder (23; 106), die ausgelegt ist, die kindersichere Sicherheitsvorrichtung (10; 100) zu der zurückgezogenen Position vorzuspannen.

3. Kindersichere Sicherheitsvorrichtung (10; 100) nach Anspruch 2, wobei die Feder (23; 106) positioniert ist, um den Deckel (14; 101) und den Griff in entgegengesetzte Richtungen vorzuspannen.

4. Kindersichere Sicherheitsvorrichtung (10; 100) nach Anspruch 2, ferner umfassend einen bewegbaren Kolben (21), der innerhalb der Schaftanordnung (16) positioniert ist, wobei der Kolben (21) gegen den Deckel (14) durch die Feder (23) vorgespannt ist.

5. Kindersichere Sicherheitsvorrichtung (10; 100) nach Anspruch 1, ferner umfassend ein Dämpfungsmittel (51) zum Dämpfen der Bewegung des Deckels (14; 101) oder des Griffs zwischen der zurückgezogenen und ausgefahrenen Position.

6. Kindersichere Sicherheitsvorrichtung (10; 100) nach Anspruch 4, wobei mindestens ein Abschnitt des Griffs innerhalb des Deckels (14) sowohl in der ausgefahrenen als auch zurückgezogenen Position positioniert ist.

7. Kindersichere Sicherheitsvorrichtung (10; 100) nach Anspruch 1, wobei das proximale Ende (16a; 104) des Griffs zum Erfassen durch einen Benutzer dient.

8. Kindersichere Sicherheitsvorrichtung (10; 100) nach Anspruch 7, wobei der lösbare Verriegelungsmechanismus (44) einen bewegbaren Ansatz auf dem Deckel (14) umfasst, die ausgelegt ist, mit dem Griff in Eingriff zu gelangen.

9. Kindersichere Sicherheitsvorrichtung (10; 100) nach Anspruch 8, wobei der Ansatz (44) einen Rippenabschnitt (58) und einen Anschlagabschnitt (60) aufweist, wobei eine Breite des Anschlagabschnitts (60) größer ist als eine Breite des Rippenabschnitts (58).

10. Kindersichere Sicherheitsvorrichtung (10; 100) nach Anspruch 9, wobei der Ansatz (44) zu der verriegelten Auslegung vorgespannt ist, in welcher der Anschlagabschnitt (60) des Ansatzes (44) gegen eine Lagerfläche (54) des Griffs anliegt, um eine Bewegung des Deckels (14; 101) oder des Griffs zu der ausgefahrenen Auslegung zu verhindern.

11. Kindersichere Sicherheitsvorrichtung (10; 100) nach Anspruch 8, wobei der Ansatz (44) einen Rippenabschnitt (58) und einen Anschlagabschnitt (60) aufweist, und der Ansatz (44) zu der verriegelten Auslegung vorgespannt ist, in welcher der Anschlagabschnitt (60) des Ansatzes (44) gegen eine Lagerfläche (54) des Griffs anliegt, um eine Bewegung des Deckels (14; 101) oder des Griffs zu der ausgefahrenen Auslegung zu verhindern.

12. Kindersichere Sicherheitsvorrichtung nach Anspruch 10, wobei der Ansatz (44) in die unverriegelte Auslegung bewegt werden kann, in welcher der Anschlagabschnitt (60) des Ansatzes (44) von der Lagerfläche (54) des Griffs getrennt ist, und der Rippenabschnitt (58) des Ansatzes (44) mit einem Schlitz (36) ausgerichtet ist, der auf dem Griff gebildet ist.

13. Kindersichere Sicherheitsvorrichtung nach Anspruch 12, wobei der Rippenabschnitt (58) ausgelegt ist, sich in dem Schlitz (36) des Griffs zu bewegen, um die Bewegung des Deckels (14; 101) oder des Griffs in die ausgefahrene Auslegung zu gestatten.

14. Kindersichere Sicherheitsvorrichtung nach Anspruch 12, wobei eine Breite des Schlitzes (36) kleiner ist als die Breite des Anschlagabschnitts (60) und größer als die Breite des Rippenabschnitts (58).

## Revendications

1. Dispositif de sécurité à l'épreuve des enfants (10; 100) pour une forme de dosage orale (12; 102), comprenant:
une poignée comprenant un ensemble d'arbre (16; 108) présentant une extrémité proximale (16a; 104) et une extrémité distale (16b) sur laquelle une forme de dosage orale (12; 102) est montée fixement soit directement soit indirectement;
un couvercle (14; 101) qui se déplace par rapport à la poignée, ou vice versa, afin de protéger sélectivement la forme de dosage orale (12; 102), dans lequel, le couvercle (14; 101) ou la poignée est poussé (e) en direction d'une position rétractée dans laquelle le couvercle (14; 101) cache la forme de dosage orale (12; 102) afin de limiter l'accès à la forme de dosage orale (12; 102), et le couvercle (14; 101) ou la poignée est déplaçable jusque dans une position déployée dans laquelle la forme de dosage orale (12; 102) est accessible à l'utilisateur; et
un mécanisme de verrouillage libérable (44; 104) qui est déplaçable entre une configuration verrouillée et une configuration déverrouillée, dans lequel, dans la configuration verrouillée, le mécanisme de verrouillage libérable (44; 104) est configuré de manière à empêcher le déplacement du couvercle (14; 101) ou de la poignée vers la configuration déployée, et, dans la configuration déverrouillée, le mécanisme de verrouillage libérable (44; 104) est configuré de manière à permettre le déplacement du couvercle (14; 101) ou de la poignée vers la configuration déployée.

2. Dispositif de sécurité à l'épreuve des enfants (10; 100) selon la revendication 1, comprenant en outre un ressort (23; 106) qui est configuré de manière à pousser le dispositif de sécurité à l'épreuve des enfants (10; 100) en direction de la position rétractée.

3. Dispositif de sécurité à l'épreuve des enfants (10; 100) selon la revendication 2, dans lequel le ressort (23; 106) est positionné de manière à pousser le couvercle (14; 101) et la poignée dans des directions opposées.

4. Dispositif de sécurité à l'épreuve des enfants (10; 100) selon la revendication 2, comprenant en outre un plongeur mobile (21) qui est positionné à l'intérieur de l'ensemble d'arbre (16), dans lequel le plongeur (21) est poussé contre le couvercle (14) par le ressort (23).

5. Dispositif de sécurité à l'épreuve des enfants (10; 100) selon la revendication 1, comprenant en outre des moyens d'amortissement (51) pour amortir le déplacement du couvercle (14; 101) ou de la poignée entre les positions rétractée et déployée.

6. Dispositif de sécurité à l'épreuve des enfants (10; 100) selon la revendication 4, dans lequel au moins une partie de la poignée est positionnée à l'intérieur du couvercle (14) à la fois dans la position déployée et dans la position rétractée.

7. Dispositif de sécurité à l'épreuve des enfants (10; 100) selon la revendication 1, dans lequel l'extrémité proximale (16a; 104) de la poignée est conçue pour être saisie par un utilisateur.

8. Dispositif de sécurité à l'épreuve des enfants (10; 100) selon la revendication 7, dans lequel le mécanisme de verrouillage libérable (44) comporte une languette déplaçable sur le couvercle (14) qui est configurée de manière à être engagée avec la poignée.

9. Dispositif de sécurité à l'épreuve des enfants (10; 100) selon la revendication 8, dans lequel la languette (44) comprend une partie de nervure (58) et une partie d'arrêt (60), dans lequel une largeur de la partie d'arrêt (60) est plus grande qu'une largeur de la partie de nervure (58).

10. Dispositif de sécurité à l'épreuve des enfants (10; 100) selon la revendication 9, dans lequel la languette (44) est poussée en direction de la configuration verrouillée dans laquelle la partie d'arrêt (60) de la languette (44) bute contre une surface d'appui (54) de la poignée afin d'empêcher le déplacement du couvercle (14; 101) ou de la poignée vers la configuration déployée.

11. Dispositif de sécurité à l'épreuve des enfants (10; 100) selon la revendication 8, dans lequel la languette (44) comprend une partie de nervure (58) et une partie d'arrêt (60), et la languette (44) est poussée en direction de la configuration verrouillée dans laquelle la partie d'arrêt (60) de la languette (44) bute contre une surface d'appui (54) de la poignée afin d'empêcher le déplacement du couvercle (14; 101) ou de la poignée vers la configuration déployée.

12. Dispositif de sécurité à l'épreuve des enfants selon la revendication 10, dans lequel la languette (44) est déplaçable vers la configuration déverrouillée dans laquelle la partie d'arrêt (60) de la languette (44) est séparée de la surface d'appui (54) de la poignée, et la partie de nervure (58) de la languette (44) est alignée avec une fente (36) qui est formée sur la poignée.

13. Dispositif de sécurité à l'épreuve des enfants selon la revendication 12, dans lequel la partie de nervure (58) est configurée de manière à se déplacer dans la fente (36) de la poignée de manière à permettre le déplacement du couvercle (14; 101) ou de la poignée jusque dans la configuration déployée.

14. Dispositif de sécurité à l'épreuve des enfants selon la revendication 12, dans lequel une largeur de la fente (36) est inférieure à la largeur de la partie d'arrêt (60) et est supérieure à la largeur de la partie de nervure (58).
